(19) Europäisches Patentamt
European Patent Office
Office européen des brevets

(11) Publication number: 0 197 661
A2

(12) # EUROPEAN PATENT APPLICATION

(21) Application number: 86301689.5

(51) Int. Cl.⁴: C 13 F 3/00

(22) Date of filing: 10.03.86

(30) Priority: 11.03.85 JP 48697/85

(43) Date of publication of application:
15.10.86 Bulletin 86/42

(84) Designated Contracting States:
BE CH DE FR GB IT LI LU NL SE

(71) Applicant: Takeda Chemical Industries, Ltd.
27, Doshomachi 2-chome Higashi-ku
Osaka-shi Osaka, 541(JP)

(72) Inventor: Matsuo, Takao
1-74, Shimotsubayashi-rokutanda
Nishikyo-ku Kyoto 615(JP)

(72) Inventor: Horii, Satoshi
32-1, Miharadai 3-cho
Sakai Osaka 590-01(JP)

(72) Inventor: Morita, Katsura
11-18, Toyoshimakita 2-chome
Ikeda Osaka 563(JP)

(74) Representative: Laredo, Jack Joseph et al,
Elkington and Fife High Holborn House 52/54 High
Holborn
London, WC1V 6SH(GB)

(54) **Low-calorie sugar composition.**

(57) A low calorie sugar composition comprising sucrose and sucrase inhibitor
(1) does not cause any side effects nor interfere with the taste of sucrose;
(2) provides the so-called "low-caloric effect" with certainty;
(3) is chemically stable;
(4) can be supplied at low cost;
(5) can be used in the same modes of use and for the same purposes as ordinary sugar.

EP 0 197 661 A2

# Low-calorie sugar composition

The present invention relates to a low-calorie sugar composition characterized by inhibited absorption from the human digestive tract.

Sugar has been used in the largest quantity as a single sweetening agent for many years. However, sugar has a high caloric value and its increased intake leads to an increased incidence of obesity and, hence, to hazardous effects on human health. Today, it is generally pointed out that the ingestion of sugar is an etiologic factor in diabetes, hypertension and other diseases.

To overcome the problem, much research work has been conducted for finding sweeteners that might simulate the sweetness of sugar but be lower in caloric value. For example, invert sugar with increased sweetness, naturally-occurring sweeteners such as stevia, and various synthetic sweetening chemicals such as saccharin, dulcin, cyclohexylaminosulfonic acid, aspartame, etc. have been developed. However, excepting the common characteristic that they are low in caloric value, these substitute sweeteners are by far inferior to sugar, for example in taste, safety and chemical stability.

On the other hand, sugar has a number of advantageous

characteristics. Thus, (i) sugar is stable against heat and light and chemically inert to other foods and food ingredients, (ii) it has no bitter taste or unsavory aftertaste even in high concentrations, (iii) it is available at low cost and in large quantities; (iv) it is highly safe to use; (v) it is colorless and odorless; (vi) it is readily soluble in water; (vii) it causes little change in the composition of concomitant chemicals; (viii) it has a long storage life; and (ix) it is crystalline and, therefore, easy to handle.

Therefore, if an excessive intake of sugar did not cause obesity nor did it adversely affect diabetic patients, sugar would after all be an ideal sweetener.

The present inventors sought for a means of reducing the caloric value of sugar without affecting its aforementioned advantages and found that the objective can be realized by using sucrose (sugar) in combination with a sucrase inhibitor.

Thus, the present invention relates to a low-calorie sugar composition which comprises sucrose and a sucrase inhibitor.

Sucrose to be employed in accordance with the present invention may be cane sugar or beet sugar or both. Sugar may be classified, according to the production process, into massecuite or centrifugal sugar; according to the degree of purification, into crude sugar or refined sugar; according to color, into white sugar, brown sugar or black sugar; and according to product shape, into powdered sugar, lump sugar, or crystal sugar, for instance, any of these types of sugar may be employed for the purposes of the present invention.

While many sucrase inhibitors are know, there are preferred those inhibitors satisfying the requirements, (i) hardly absorbed from the digestive tract, (ii) not

jeopardizing the advantages of sugar, particularly the taste of sugar, and (iii) substantially harmless to humans. In addition, preferred are those having comparatively high sucrase-inhibiting activity.

As examples of such sucrase inhibitors, there may be mentioned the valiolamine derivatives described in European Patent Publication No. 56194 and 89812. These valiolamine derivatives have the general formula:

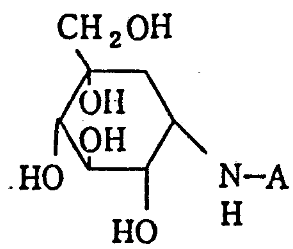

[I]

[wherein A is an acyclic hydrocarbon group of 1 to 10 carbon atoms which may have one or more substituents, whether the same or different selected from the class consisting of hydroxy, phenoxy, thienyl, furyl, pyridyl, cyclohexyl, and substituted or unsubstituted phenyl, a 5- to 6- membered cyclic hydrocarbon group which may have one or more substituents, whether the same or different selected from the class consisting of hydroxy, hydroxymethyl, methyl and amino, or a saccharide residue].

Specific examples of N-substituted valiolamine derivatives having the above general formula [I] include the following.

(1) N-Phenethylvaliolamine
(2) N-(3-Phenylallyl)valiolamine
(3) N-Furfurylvaliolamine
(4) N-Thenylvaliolamine
(5) N-(3-Pyridylmethyl)valiolamine
(6) N-(4-Bromobenzyl)valiolamine

(7) N-[(R)-β-Hydroxyphenethyl]valiolamine
(8) N-[(S)-β-Hydroxyphenethyl]valiolamine
(9) N-(β-Hydroxy-2-methoxyphenethyl]valiolamine
(10) N-(3,5-Di-tert-butyl-4-hydroxybenzyl)valiolamine
(11) N-(Cyclohexylmethyl)valiolamine
(12) N-Geranylvaliolamine
(13) N-(1,3-Dihydroxy-2-propyl)valiolamine
(14) N-(1,3-Dihydroxy-1-phenyl-2-propyl)valiolamine
(15) N-[(R)-α-(Hydroxymethyl)benzyl]valiolamine
(16) N-Cyclohexylvaliolamine
(17) N-(2-Hydroxycyclohexyl)variolamine
(18) N-[(1R,2R)-2-Hydroxycyclohexyl]valiolamine
(19) N-(2-Hydroxycyclopentyl)valiolamine
(20) Methyl 4-[(1S,2S)-(2,4,5(OH)/3,5)-2,3,4,5-tetrahydroxy-5-(hydroxyxmethyl)cyclohexyl]amino-4,6-dideoxy-α-D-glucopyranoside
(21) Methyl 4-[(1S,2S)-(2,4,5(OH)/3,5)-2,3,4,5-tetrahydroxy-5-(hydroxyxmethyl)cyclohexyl]amino-4-deoxy-α-D-glucopyranoside
(22) [(1S,2S)-(2,4,5(OH)/3,5)-2,3,4,5-Tetrahydroxy-5-(hydroxymethyl)cyclohexyl][(1R,2S)-(2,6/3,4)-4-amino-2,3-dihydroxy-6-(hydroxymethyl)cyclohexyl]-amine
(23) N-[(1R,2S)-(2,4/3,5)-2,3,4-Trihydroxy-5-(hyhydroxymethyl)cyclohexyl]valiolamine
(24) N-[(1R,2S)-(2,6/3,4)-4-Amino-2,3-dihydroxy-6-methylcyclohexyl]valiolamine
(25) N-[(1R,2S)-(2,6/3,4)-2,3,4-Trihydroxy-6-methylcyclohexyl]valiolamine
(26) N-[(1R,2S)-(2,4,6/3)-2,3,4-Trihydroxy-6-methylcyclohexyl]valiolamine
(27) 4-O-α-[4-[((1S)-(1,2,4,5(OH)/3,5)-2,3,4,5-Tetrahydroxy-5-(hydroxymethyl)cyclohexyl)amino]-4,6-dideoxy-D-glucopyranosyl]-D-glucopyranose
(28) 1,6-Anhydro-4-O-α-[4-[((1S)-(1,2,4,5(OH)/3,5)-2,3,4,5-tetrahydroxy-5-(hydroxymethyl)cyclohexyl)amino]-4,6-

dideoxy-D-glucopyranosyl]-β-D-glucopyranose

The N-substituted valienamine derivatives described in European Patent Publication No. 49981, which have the general formula:

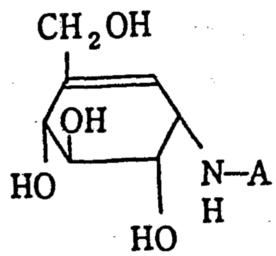

[II]

[wherein A is an acyclic hydrocarbon group of 1 to 10 carbon atoms which may have one or more substituents, whether the same or different selected from the class consisting of hydroxy, phenoxy, thienyl, furyl, pyridyl, cyclohexyl, and substituted or unsubstituted phenyl, a 5- or 6-membered cyclic hydrocrbon group which may have one or more substituents, whether the same or different, selected from the class consisting of hydroxy, hydroxymethyl, methyl and amino, or a saccharide residue] and the N-substituted validamine derivatives described in European Patent Publication No. 56194 which have the general formula:

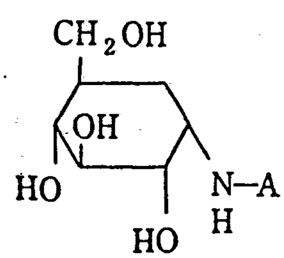

[III]

[wherein A has the same meaning as A in general formula [II]] can also be used with advantage as the sucrase inhibitor according to the present invention.

The proportions of components in the sugar composition according to the present invention may be chosen in

accordance with the particular species of sucrase inhibitor employed but as long as the effect of the present invention can be realized, the proportion of the sucrase inhibitor is preferably smaller, for the organoleptic and other qualities of sugar are then not much sacrificed. Though the ratio varies with different species of sucrase inhibitors, it is generally desirable that, for each 100 weight parts of sucrose, $1.0 \times 10^{-5}$ to $1.0 \times 10^{-1}$ weight part, preferably $5.0 \times 10^{-5}$ to $5.0 \times 10^{-2}$ weight part, and for still better results, $1.0 \times 10^{-4}$ to $4.0 \times 10^{-3}$ weight part of the sucrase inhibitor be employed. Particularly in regard to the inhibitor compounds of the general formula [I], it is preferable to use $5.0 \times 10^{-5}$ to $4.0 \times 10^{-3}$ weight part, for still better results, $1.0 \times 10^{-4}$ to $2.0 \times 10^{-3}$ weight part.

The composition of the present invention can be produced by admixing sucrose evenly with said sucrase inhibitor. Or one may use a process in which a solution of the sucrase inhibitor is mixed with solid state sucrose or a solution of sucrose and the mixture is spray-dried or otherwise dried to separate out the desired composition. One may also use a process in which a solution of the sucrase inhibitor is sprayed over solid sugar and dried as such. In any of such processes, it is important to ensure that the inhibitor is evenly blended with the sucrose.

An exemplary process for producing the sugar composition of the present invention will now be described in detail.

Sucrose is dissolved in a minimum volume of hot water, while the inhibitor is dissolved in water or an aqueous organic solution such as aqueous ethanol. The two solutions are mixed and concentrated under reduced pressure to crystallize the sucrose. In the operation, the inhibitor is evenly dispersed in sucrose crystals.

In another preferred process, sucrose either as a free-flowing powder, in an atomized liquid state or in the

form of a moving thin layer, is sprayed with a solution of the sucrase inhibitor in aqueous ethanol and, then dried.

The composition thus produced is supplemented with additives as needed or/and milled or molded to provide the desired final product.

The additives that can be incorporated in the composition of the present invention include those food additives which are commonly added to foods, such as flavors, colors, vitamins and so on.

The composition according to the present invention can be used in the conventional applications for food-grade sugar. For example, it can be used as table sugar or in cooking applications. It can also be added to liquid or solid foods, seasonings, delicacies, etc., such as nonalcoholic beverages, fruit juices, milk, jams, bean jams, jellies, confections, corn flakes, and so on.

When the composition according to the present invention was orally administered to animals, the absorption of sucrose from the digestive tract was markedly inhibited and, moreover, no adverse effects were found at all. Thus, most of the administered sucrose was not digested and absorbed in the small intestine but some was utilized by the indigenous bacteria in the large intestine and some was excreted into feces via the large intestine, causing substantially no elevation of blood glucose level. This finding was duplicated in humans. The sucrose that reached the large intestine was partially utilized for growth of intestinal bacteria but since the intestinal bacteria are useful for in vivo synthesis of vitamins, this feature is also a favorable aspect of the present invention.

Thus, the composition according to the present invention has a number of advantageous features. For example, (1) as it contains only a small amount of sucrase inhibitor, the composition does not cause side effects nor

does it interfere with the taste of the sucrose, (2) it provides the so-called "low-calorie effect" with certainty; (3) it is chemically stable and withstands prolonged storage; (4) it can be produced at low cost; (5) it can be used in the same modes of use and for the same purposes as ordinary sugar; and (6) it is easy to handle.

Example 1

In 100 weight parts of hot water was dissolved 100 weight parts of coarse granulated sugar to provide a sucrose solution. On the other hand, 3.0 x $10^{-3}$ weight part of the sucrase inhibitor N-(1,3-dihydroxy-2-propyl)valiolamine, i.e. 1L(1S) (1(OH),2,4,5/1,3)-5-[(2-hydroxy-1-(hydroxymethyl)-ethyl]amino]-1-C-(hydroxymethyl)-1,2,3,4-cyclohexanetetrol (hereinafter referred to as AO-128), was dissolved in 1 weight part of water. The two solutions were mixed and concentrated under reduced pressure. When most of the water had been distilled off, crystallization occurred. The water was further distilled off and the residue was dried to give a solid product.

Example 2

In 10 weight parts of 50% aqueous ethanol was dissolved 0.04 weight part of the same sucrase inhibitor as used in Example 1. Then, with 100 weight parts of granular sugar spread out in a thin layer, the above solution was sprayed over the layer from a spray nozzle, followed by drying under reduced pressure. After addition of a trace amount of lemon flavor, the product was milled to provide a sugar composition for black tea.

Example 3

In 100 volume parts of water-ethanol (70:30 by volume) was dissolved 100 weight parts of granular sugar and 1.0 x $10^{-3}$ weight part of the same sucrase inhibitor as used in Example 1 was dissolved. Then, the solution was worked up in the same manner as Example 1 to provide a powdery

composition. In this example, concentration and drying could be performed more easily than in Example 1.

Example 4

One-hundred weight parts of refined white sugar and 1.5 x $10^{-3}$ weight part of the same sucrase inhibitor as used in Example 1 were dissolved in water at 80°C to provide 1 liter of a sucrose solution. This solution was spray-dried by means of a spray dryer (produced by Ohkawara Kakoki, in Japan) to give a powdery composition.

When the composition according to the present invention was orally administered to animals, elevation of blood glucose was significantly inhibited as compared with plain sucrose without addition of the sucrase inhibitor. The tests and their results are shown below.

Experiment

Test Conditions

Male rats (Jcl:SD strain, purchased from Clea Japan) aged 6 weeks, which had been raised on commercially available pelleted rat chow (CE-2®, Clea Japan), were fasted for 20 hours and, then, administered orally with 2.5 g/kg of sucrose containing a varying dosage of AO-128. The blood glucose level was determined 30, 60 and 120 minutes after administration.

Table 1

Test Results

| Test Group | Blood glucose level mg/dℓ | | | | Delta glucose area (mg/dℓ·hr) |
|---|---|---|---|---|---|
| | 0 min. | 80 min. | 60 min. | 120 min. | |
| Control | 71±4 | 144±6 | 127±8 | 108±1 | 95±7 |
| AO-128, 0.1mg/Kg | 69±4 | 106±8 a | 101±2 a | 88±3 a | 52±5 b |
| 〃 0.2mg/Kg | 74±8 | 102±6 b | 90±5 b | 88±4 b | 38±7 a |
| 〃 0.4mg/Kg | 67±4 | 79±2 a | 77±5 a | 71±5 a | 15±9 a |

Mean ± Standard deviation

Significant difference from control (a:$P<0.001$, b:$P<0.01$)

The composition according to the present invention is highly safe and the oral administration of the composition which had been confirmed to be useful in animal experiments to human subjects did not cause elevation of blood glucose just as in animals.

What is claimed is:

1. A low-calorie sugar composition comprising sucrose and a sucrase inhibitor.

2. A composition claimed in Claim 1 wherein said sucrase inhibitor is a valiolamine derivative of the general formula:

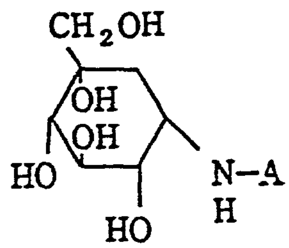

[wherein A is an acyclic hydrocarbon group of 1 to 10 carbon atoms which may have one or more substituents, whether the same or different, selected from the class consisting of hydroxy, phenoxy, thienyl, furyl, pyridyl, cyclohexyl, and substituted or unsubstituted phenyl, a 5- to 6- membered cyclic hydrocarbon group which may have one or more substituents, whether the same or different, selected from the class consisting of hydroxy, hydroxymethyl, methyl and amino, or a saccharide residue].

3. A composition claimed in claim 1 wherein said sucrase inhibitor is N-(1,3-dihydroxy-2-propyl)-valiolamine.

4. A composition claimed in claim 1 wherein the proportion of the sucrase inhibitor to 100 weight parts of the sucrose is $1.0 \times 10^{-5}$ to $1.0 \times 10^{-1}$ weight part.

5. A composition claimed in claim 3 wherein the proportion of the sucrase inhibitor to 100 weight parts of the sucrose is $5.0 \times 10^{-5}$ to $4.0 \times 10^{-3}$ weight part.